(19) Europäisches Patentamt
European Patent Office
Office européen des brevets

(11) Veröffentlichungsnummer: **0 478 990 B1**

# (12) EUROPÄISCHE PATENTSCHRIFT

(45) Veröffentlichungstag der Patentschrift: **28.06.95**

(51) Int. Cl.$^{6}$: **C07D 229/00**, C07D 251/34, C08G 18/79, C09D 175/04

(21) Anmeldenummer: **91115187.6**

(22) Anmeldetag: **09.09.91**

(54) **Polyisocyanatgemisch, ein Verfahren zu seiner Herstellung und seine Verwendung in Polyurethanlacken.**

(30) Priorität: **20.09.90 DE 4029809**

(43) Veröffentlichungstag der Anmeldung:
**08.04.92 Patentblatt 92/15**

(45) Bekanntmachung des Hinweises auf die Patenterteilung:
**28.06.95 Patentblatt 95/26**

(84) Benannte Vertragsstaaten:
**AT BE CH DE ES FR GB IT LI NL SE**

(56) Entgegenhaltungen:
**EP-A- 0 047 452**
**EP-A- 0 166 173**
**DE-A- 1 934 763**
**GB-A- 1 153 815**

(73) Patentinhaber: **BAYER AG**

**D-51368 Leverkusen (DE)**

(72) Erfinder: **Laas, Hans Josef, Dr.**
**Merheimer Strasse 249**
**W-5000 Köln 60 (DE)**
Erfinder: **Halpaap, Reinhard, Dr.**
**In der Hildscheid 6**
**W-5068 Odenthal-Glöbusch (DE)**
Erfinder: **Pedain, Josef, Dr.**
**Haferkamp 6**
**W-5000 Köln 80 (DE)**
Erfinder: **Mosbach, Jürgen, Dr.**
**Katterbachstrasse 108**
**W-5060 Bergisch Gladbach (DE)**

**EP 0 478 990 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues, im wesentlichen aus Uretdiondiisocyanaten bestehendes Polyisocyanatgemisch auf Basis von Gemischen von 1,6-Diisocyanatohexan (HDI) und 1-Isocyanato-3,3,5-trimethyl-5-isocyanatomethylcyclohexan (IPDI) und deren Verwendung, gegebenenfalls in mit Blockierungsmitteln blockierter Form, in Polyurethanlacken.

Die Herstellung von Uretdiongruppen aufweisenden Polyisocyanaten durch teilweise Dimerisierung aliphatischer Diisocyanate in Gegenwart geeigneter Katalysatoren ist aus einer Reihe von Veröffentlichungen bekannt.

Die DE-OS 3 030 513 beschreibt die Dimerisierung von IPDI, wobei Tris-(dialxylamino)-phosphine als Katalysator verwendet werden. Die Uretdione von 2-Methyl-1,5-diisocyanatopentan und 2-Ethyl-1,4-diisocyanatobutan lassen sich nach der DE-OS 3 227 779 auf dem gleichen Wege herstellen. Das Verfahren der DE-OS 3 437 635 gestattet bei Verwendung von H-aktiven organischen Co-Katalysatoren, wie Alkoholen oder Aminen, auch die Dimerisierung anderer aliphatischer Diisocyanate, wie z.B. HDI. Die bekanntermaßen sehr leicht eintretende Oxidation des bei diesen Verfahren bevorzugt als Katalysator eingesetzten Tris-(dimethylamino)-phosphins mit Luftsauerstoff zum cancerogenen Hexamethylphosphorsäuretriamid macht jedoch deren praktische Anwendung bedenklich.

In den DE-OSen 1 670 720 und 3 420 114 werden Lewis-Säuren wie Bortrifluorid bzw. Antimonpentafluorid als Dimerisierungskatalysatoren für aliphatische Diisocyanate vorgeschlagen. Diese Verfahren konnten sich u.a. aufgrund der stark korrosiven Wirkung dieser Katalysatoren nicht durchsetzen.

Nach der DE-OS 3 739 549 sind dialkylaminosubstituierte Pyridine wie p-Dimethylaminopyridin (DMAP) geeignete Katalysatoren zur Herstellung reiner Uretdione aus aliphatischen Diisocyanaten. Nach eigenen Versuchen weisen die nach diesem Verfahren aus HDI und IPDI gewonnenen Produkte jedoch eine starke Eigenfärbung auf, was ihrer breiten Anwendung als Lackpolyisocyanate entgegensteht.

Die am längsten bekannte Methode zur Dimerisierung aliphatischer Diisocyanate besteht in ihrer Modifizierung mit Hilfe tertiärer Phosphine, bevorzugt mit Trialkylphosphinen. Dies ist Gegenstand der DE-OSen 1 670 720 und 1 934 763 sowie der US-PS 4 614 785. Die auf diese Weise erhaltenen Produkte weisen neben Uretdiongruppen immer einen mehr oder weniger großen Anteil an Isocyanuratgruppen auf und sind relativ dunkel gefärbt. Es wurden jedoch laufend weitere Versuche zur Verbesserung des Verfahrens unternommen. Durch Urethanisierung eines Teils der Isocyanatgruppen mit geeigneten Alkoholen vor oder während der Oligomerisierungsreaktion und Behandeln des nach Dünnschichtdestillation anfallenden Harzes mit organischen Peroxiden lassen sich, wie in DE-OS 3 900 053 beschrieben, ausgehend von HDI niedrigviskose, uretdiongruppenhaltige Polyisocyanate mit geringer Farbzahl herstellen. Diese eignen sich insbesondere für den Einsatz in lichtechten Ein- oder Zweikomponentenpolyurethanlacken, können dabei aber nicht allen Anforderungen der Praxis gerecht werden.

Lackpolyisocyanate auf Basis von HDI, insbesondere auch Uretdiongruppen aufweisende Diisocyanate auf Basis von HDI sind den entsprechenden Polyisocyanaten auf Basis von IPDI bezüglich der Wetterresistenz der aus den Polyisocyanaten erhaltenen Überzüge unterlegen. Im übrigen eignen sich die Uretdiongruppen aufweisenden Diisocyanate auf Basis von HDI aufgrund ihrer niedrigen Viskosität z.B. nicht als Ausgangsmaterial zur Herstellung von Pulverlacken. Hierfür eignen sich jedoch Uretdiongruppen aufweisende Diisocyanate auf Basis von IPDI (vgl. z.B. EP-A-0 045 998). Der Nachteil dieser Uretdiondiisocyanate ist jedoch in ihrer schlechten Zugänglichkeit zu sehen, da die Dimerisierung von IPDI mit den zur Dimerisierung von aliphatischen Diisocyanaten bevorzugt eingesetzten Trialkylphosphin-Katalysatoren relativ hohe Katalysatorkonzentrationen (1 bis 3 %) und sehr lange Reaktionszeiten (8 Tage bis 8 Wochen) benötigt (DE-OS 1 934 763).

Es war daher die der Erfindung zugrundeliegende Aufgabe, neue, Uretdiongruppen aufweisende Polyisocyanate zur Verfügung zu stellen, die zu einem beträchtlichen Teil, vorzugsweise überwiegend aus dimerisiertem IPDI bestehen, und die demzufolge die von Lackpolyisocyanaten auf IPDI-Basis bekannten guten lacktechnischen Eigenschaften in sich vereinigen und gleichzeitig in hohen Raum-Zeit-Ausbeuten herstellbar sind.

Diese Aufgabe konnte mit dem nachstehend näher beschriebenen erfindungsgemäßen Verfahren zur Herstellung der neuen erfindungsgemäßen Polyisocyanatgemische gelöst werden. Das nachstehend näher beschriebene erfindungsgemäße Verfahren basiert auf der überraschenden Beobachtung, daß sich aus Mischungen von HDI und IPDI mit schon vergleichsweise geringem Gehalt an HDI durch Modifizierung mit Trialkylphosphinen sehr leicht uretdiongruppenhaltige Polyisocyanate herstellen lassen, in denen ein sehr hoher Anteil des cycloaliphatischen Diisocyanats eingebaut ist.

Auf diese Weise wird selbst die Herstellung hochviskosr bis fester Produkte unter praxisgerechten Bedingungen, d.h. mit geringen Katalysatorkonzentrationen und relativ kurzen Reaktionszeiten, ermöglicht.

Obwohl sich in einigen der obengenannten Veröffentlichungen, z.B. in der US-PS 4 614 785, den DE-OSen 3 420 114, 3 739 549 oder 3 900 053 nach Aufzählung langer Listen geeigneter (cyclo)-aliphatischer Diisocyanate auch der Hinweis findet, daß auch Gemische dieser Isocyanate zur Dimerisierung eingesetzt werden können, konnte der Fachmann diesem Hinweis keineswegs entnehmen, daß sich durch Beimischung von HDI zu IPDI die Reaktionszeiten zur Herstellung von Uretdionen selbst bei sehr hohem Gehalt an IPDI in der Ausgangsmischung drastisch verkürzen lassen und IPDI in großer Menge in das Polyisocyanat eingebaut wird, wie dies aus dem nachstehenden experimentellen Befund hervorgeht. Dies ist nach dem oben geschilderten Stand der Technik überraschend, da aufgrund des großen Reaktivitätsunterschiedes zwischen HDI und IPDI zu erwarten war, daß sich aus Mischungen der beiden Diisocyanate vorzugsweise das reine HDI-Uretdion und nicht ein Mischoligomerisat mit hohem IPDI-Anteil bilden würde.

Die katalytische Oligomerisierung von HDI/IPDI-Gemischen ist auch Gegenstand der EP-A-0 047 452, jedoch werden in dieser Vorveröffentlichung ausschließlich Mischtrimerisate der genannten Diisocyanate beschrieben, die praktisch frei von Uretdiongruppen sind.

Gegenstand der Erfindung sind Polyisocyanatgemische mit einem Uretdiongehalt (berechnet als $C_2N_2O_2$) von 8 bis 25 Gew.-% und einem Isocyanuratgehalt (berechnet als $C_3N_3O_3$) von max. 24 Gew.-%, bestehend im wesentlichen aus

a) 20 bis 60 Gew.-% Uretdiondiisocyanat der Formel (I)

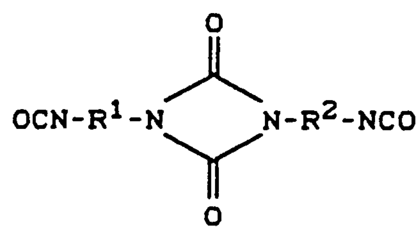

,

b) 0 bis 40 Gew.-% Isocyanurattriisocyanat der Formel (II)

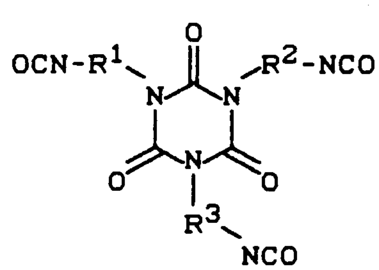

,

c) 15 bis 60 Gew.-% an höheren, mehr als einen Uret aufweisenden Homologen der Polyisocyanate I und/oder II und

d) insgesamt maximal 1 Gew.-% 1,6-Diisocyanatohexan (HDI) und 1-Isocyanato-3,3,5-trimethyl-5-isocyanatomethyl-cyclohexan (IPDI),

wobei sich die genannten Prozentsätze zu 100 ergänzen, und wobei

$R^1$, $R^2$ und $R^3$ gleich oder verschieden sind und für die die Isocyanatgruppen von HDI oder IPDI verknüpfenden Kohlenwasserstoffreste stehen, wobei im letztgenannten Fall die Isocyanatgruppen mit dem cycloaliphatischen Ring verknüpft sind, mit der Maßgabe, daß mindestens 15 Mol-% und höchstens 95 Mol-% der genannten Reste Hexamethylenreste darstellen.

Gegenstand der Erfindung ist auch die Verwendung der neuen Polyisocyanatgemische, gegebenenfalls in mit Blockierungsmitteln für Isocyanatgruppen blockierter Form, als Isocyanatkomponente in Polyurethanlacken.

Ausgangsmaterialien für das erfindungsgemäße Verfahren sind HDI und IPDI, die im Molverhältnis von 1:9 bis 9:1, vorzugsweise von 1:7 bis 7:1 zum Einsatz gelangen.

Zur Herstellung der erfindungsgemäßen Dimeren besonders gut geeignete Katalysatoren sind tertiäre organische Phosphine, wie sie beispielsweise in der US-PS 4 614 785, Kolonne 4, Zeilen 11 bis 47,

beschrieben sind. Bevorzugte tertiäre Phosphine sind Tri-n-butylphosphin und Tri-n-octylphosphin. Selbstverständlich kommen als Katalysatoren für das erfindungsgemäße Verfahren auch alle sonstigen an sich bekannten Dimerisierungskatalysatoren in Betracht.

Als Beispiele für derartige Dimerisierungskatalysatoren seien die in den DE-OSen 3 030 513, 3 227 779 und 3 437 635 genannten Tris-(dialkylamino)-phosphine genannt, Antimonpentafluorid nach der DE-OS 3 420 114, Bortrifluorid gemäß DE-OS 1 670 720 sowie p-Dimethylaminopyridin nach der DE-OS 3 739 549.

Die Dimerisierungskatalysatoren werden im allgemeinen in Mengen von 0,01 bis 5, vorzugsweise 0,1 bis 3 Gew.-%, bezogen auf das Ausgangsisocyanatgemisch, eingesetzt. Bei Verwendung von tertiären Phosphinen als Katalysatoren werden diese im allgemeinen in einer Menge von 0,1 bis 5, vorzugsweise 0,2 bis 2 Gew.-%, bezogen auf eingesetztes Diisocyanatgemisch, eingesetzt.

Neben diesen Katalysatoren können erfindungsgemäß auch Cokatalysatoren eingesetzt werden. Hierfür eignen sich niedermolekulare, ein- oder mehrwertige Alkohole, d.h. insbesondere solche des Molekulargewichtsbereichs 32 bis 200 bzw. beliebige Gemische derartiger Alkohole. Geeignet sind beispielsweise Methanol, Ethanol, n-Propanol, Isopropanol, n-Butanol, n-Hexanol, 2-Ethyl-1-hexanol, Ethylenglykol, Propylenglykol, die isomeren Butandiole, Hexandiole oder Octandiole, Diethylenglykol, Dipropylenglykol, Glycerin, Trimethylolpropan oder beliebige Gemische derartiger Alkohole.

Diese Cokatalysatoren werden, falls überhaupt, in Mengen von 0,1 bis 5, vorzugsweise 0,5 bis 3 Gew.-%, bezogen auf das Ausgangsisocyanatgemisch, eingesetzt.

Zur Reaktionsstoppung geeignete Katalysatorengifte stellen beispielsweise Alkylierungsmittel wie Dimethylsulfat oder p-Toluolsulfonsäuremethylester` Acylierungsmittel wie Benzoylchlorid, Säuren wie Perfluorbutansulfonsäure, aber auch Schwefel oder Sulfonylisocyanate dar, wie sie in der US-PS 4 614 785, Kolonne 5, Zeile 27 bis Kolonne 6, Zeile 35 beispielhaft genannt sind. Die zur Abstoppung der Reaktion benötigte Menge des Katalysatorgiftes richtet sich nach der Menge des verwendeten Katalysators; im allgemeinen wird eine äquimolare Menge des Abstoppers, bezogen auf den zu Beginn der Reaktíon vorliegenden Dimerisierungskatalysator, eingesetzt. Berücksichtigt man allerdings eventuell während der Reaktion auftretende Katalysatorverluste, so können zur Reaktionsstoppung auch schon 20 bis 80 Äquivalent-% des Katalysatorgiftes, bezogen auf die ursprünglich eingesetzte Katalysatormenge, ausreichen.

Die Herstellung der erfindungsgemäßen Dimeren kann in Substanz oder auch in Gegenwart von gegenüber Isocyanatgruppen inerten Lösungsmitteln durchgeführt werden. Geeignete Lösungsmittel sind beispielsweise Hexan, Toluol, Xylol, Chlorbenzol, Essigsäureethylester, Essigsäurebutylester, Ethylglykolacetat, Propylenglykolmonomethyletheracetat, Aceton, Methylisobutylketon, Methylenchlorid, N-Methylpyrrolidon oder beliebige Gemische derartiger Lösungsmittel.

Zur Herstellung der erfindungsgemäßen Dimeren wird eine Mischung der Ausgangsdiisocyanate, gegebenenfalls unter Inertgas wie beispielsweise Stickstoff und gegebenenfalls in Gegenwart eines geeigneten Lösungsmittels der beispielhaft genannten Art auf eine Temperatur zwischen 20 und 100 °C, vorzugsweise 40 bis 70 °C gebracht. Dann kann der gegebenenfalls mitzuverwendende alkoholische Cokatalysator zugemischt werden. Im Anschluß hieran wird, gegebenenfalls nach Beendigung der spontan ablaufenden Urethanisierungsreaktion, ein Dimerisierungskatalysator der beispielhaft genannten Art in der oben angegebenen Menge zugegeben und die Reaktionstemperatur durch eine geeignete Maßnahme (Heizen oder Kühlen) auf eine Temperatur innerhalb des Temperaturbereichs von 40 bis 120 °C, vorzugsweise von 50 bis 80 °C eingestellt. Im allgemeinen wird die Umsetzung bei Erreichen eines Oligomerisierungsgrads von 10 bis 60 %, vorzugsweise von 10 bis 40 % beendet. Unter "Oligomerisierungsgrad" ist der Prozentsatz der im Ausgangsgemisch vorliegenden Isocyanatgruppen zu verstehen, der während der erfindungsgemäßen Umsetzung (insbesondere durch Dimerisierung, daneben unter Trimeriierung und im Falle der Mitverwendung von alkoholischen Cokatalysatoren unter Urethanisierung) verbraucht wird. Der genannte Oligomerisierungsgrad wird im allgemeinen nach einer Reaktionszeit von 1 bis 48, vorzugsweise 2 bis 24 Stunden erreicht. Die Beendigung der Umsetzung kann durch Zugabe eines Katalysatorengifts der beispielhaft genannten Art und/oder durch kurzzeitiges Erhitzen der Reaktionsmischung auf oberhalb 80 °C, vorzugsweise oberhalb 120 °C liegende Temperaturen abgebrochen werden.

Anschließend wird das Reaktionsgemisch im allgemeinen im Hochvakuum, bevorzugt im Dünnschichtverdampfer, von flüchtigen Bestandteilen (überschüssigen Monomeren und gegebenenfalls mitverwendeten Lösungsmitteln) befreit, wobei man als Destillationsrückstand die erfindungsgemäßen Polyisocyanatgemische erhält. Die so erhaltenen erfindungsgemäßen Polyisocyanatgemische stellen praktisch farblose Polyisocyanate dar, deren Konsistenz vor allem vom Verhältnis HDI/IPDI abhängt.

Aus der molaren Zusammensetzung des wiedergewonnenen Diisocyanatgemisches kann man das Verhältnis von HDI- und IPDI-Resten in den erfindungsgemäßen Gemischen rechnerisch bestimmen. Es zeigt sich, daß sich unter den Bedingungen des erfindungsgemäßen Verfahrens HDI etwas reaktiver als IPDI verhält, weshalb sich das Verhältnis von HDI- zu IPDI-Resten in den erfindungsgemäßen Gemischen, im

Vergleich zur Zusammensetzung der Ausgangsmischung, zugunsten des Hexamethylenrestes verschiebt. Dies geschieht in Abhängigkeit von Reaktionsbedingungen wie Temperatur, Katalysatorkonzentration oder Oligomerisierungsgrad.

Die Konsistenz der erfindungsgemäßen Dimeren hängt überwiegend vom Verhältnis der Diisocyanatreste ab. Produkte mit einem HDI-Anteil von weniger als 40 Gew.-% sind im allgemeinen hochviskos, während solche mit höherem HDI-Anteil bei Raumtemperatur flüssig sind. Die erfindungsgemäßen Polyisocyanatgemische mit hohem IPDI-Anteil, die bei 23 °C eine Viskosität von mindestens 50.000 mPa.s aufweisen, stellen oftmals in mit geeigneten Blockierungsmitteln, insbesondere mit $\epsilon$-Caprolactam blockierter Form bei Raumtemperatur feste Substanzen dar, die sich als Pulverlack-Vernetzer eignen.

Der NCO-Gehalt der erfindungsgemäßen Produkte hängt ebenfalls von deren Zusammensetzung ab und liegt im allgemeinen zwischen 15 und 23 Gew.-%, vorzugsweise zwischen 17 und 21 Gew.-%.

Aus der Zusammensetzung der erfindungsgemäßen Produkte sowie aus der Tatsache, daß IPDI unter den Bedingungen des erfindungsgemäßen Verfahrens praktisch nicht dimerisiert werden kann (Vergleichsversuch), kann gefolgert werden, daß es sich bei den erfindungsgemäßen Produkten um überwiegend echte Mischdimerisate entsprechend der oben angegebenen Formel handelt, was aber selbstverständlich das Vorliegen von "Homooligomerisaten" der beiden Diisocyanate nicht ausschließt.

Hieraus und aus gelchromatographischen Untersuchungen geht hervor, daß die erfindungsgemäßen Polyisocyanatgemische die bereits obengenannte Zusammensetzung aufweisen. Die bevorzugten erfindungsgemäßen Polyisocyanatgemische weisen einen NCO-Gehalt von 17 bis 21 Gew.-%, einen Uretdiongehalt ($C_2N_2O_2$) von 10 bis 20 Gew.-% und einen Isocyanuratgehalt ($C_3N_3O_3$) von 0 bis 20 Gew.-% auf und bestehen

a) zu 25 bis 55 Gew.-% aus Uretdiondiisocyanaten der obengenannten Formel (I),

b) zu 0 bis 30 Gew.-% aus Trisisocyanato-monoisocyanuraten der obengenannten Formel (II),

c) zu 20 bis 55 Gew.-% aus höheren, mehr als einen Uretdion- und/oder mehr als einen Isocyanuratring aufweisenden Homologen der unter a) und b) genannten Polyisocyanate und

d) zu insgesamt maximal 0,5 Gew.-% monomerem HDI und/oder IPDI.

Die erfindungsgemäßen Gemische stellen wertvolle Ausgangsmaterialien zur Herstellung von Polyurethankunststoffen nach dem Polyadditionsverfahren, insbesondere zur Herstellung von Ein- oder Zwei-Komponenten-Polyurethanlacken, sowie in mit an sich bekannten Blockierungsmitteln blockierter Form wertvolle Ausgangsmaterialien für Zweikomponenten-Polyurethan-Einbrennlacke dar.

Bevorzugte Reaktionspartner für die erfindungsgemäßen, gegebenenfalls in blockierter Form vorliegenden Gemische bei der Herstellung von Polyurethanlacken sind die in der Polyurethanchemie an sich bekannten Polyhydroxypolyester und -ether, Polyhydroxypolyacrylate, Polycarbonsäuren und gegebenenfalls niedermolekulare, mehrwertige Alkohole. Auch Polyamine, insbesondere in blockierter Form als Polyketimine oder Oxazolidine sind brauchbare Reaktionspartner für die erfindungsgemäßen Mischpolymerisate.

Die Mengenverhältnisse werden im allgemeinen so gewählt, daß auf eine, gegebenenfalls blockierte, Isocyanatgruppe 0,8 bis 3, vorzugsweise 0,9 bis 1,1. Hydroxy-, Amino- und/oder Carboxylgruppen entfallen.

Zur Beschleunigung der Aushärtung können in bekannter Weise die in der Isocyanatchemie üblichen Katalysatoren verwendet werden, wie z.B. tert. Amine wie Triethylamin, Pyridin, Methylpyridin, Benzyldimethylamin, N,N-Endoethylenpiperazin, N-Methylpiperidin, Pentamethyldiethylentriamin, N,N-Dimethylaminocyclohexan, N,N'-Dimethylpiperazin usw., Metallsalze wie Eisen(III)-chlorid, Zinkchlorid, Zink-2-ethylcaproat, Zinn(II)-ethylcaproat, Dibutylzinn(IV)-dilaurat, Molybdänglykolat usw.

Bei Verwendung der erfindungsgemäßen Gemische in Einbrennlacken werden deren NCO-Gruppen ganz oder teilweise in bekanter Weise blockiert. Hierzu wird das Polyisocyanat mit einem geeigneten Blockierungsmittel, vorzugsweise bei erhöhter Temperatur, gegebenenfalls in Gegenwart eines geeigneten Katalysators (s.o.) umgesetzt.

Geeignete Blockierungsmittel sind beispielsweise Monophenole (Phenol, Kresole), tertiäre Alkohole, (t.-Butanol, Dimethylphenylcarbinol), leicht enolisierbare Verbindungen (Acetessigester, Malonsäurederivate), sekundäre, aromatische Amine (N-Methylanilin, N-Phenylxylidin), Imide (Succinimid), Lactame ($\epsilon$-Caprolactam, $\delta$-Valerolactam), Oxime (Butanonoxim, Cyclohexanonoxim), Mercaptane (Methylmercaptan, Ethylmercaptan), Triazole (1H-1,2,4-triazol).

Zur Herstellung der Lackbindemittel werden beispielsweise gegebenenfalls blockiertes Polyisocyanat, polyfunktionelle Reaktionspartner, Katalysator und gegebenenfalls die üblichen Zusätze, wie z.B. Pigmente, Füll- und Farbstoffe und Verlaufmittel miteinander auf einem üblichen Mischaggregat, wie z.B. auf einer Sandmühle entweder mit oder ohne Lösungs- und Verdünnungsmittel gut vermischt und homogenisiert.

Als Lösungsmittel geeignet sind alle an sich bekannten üblichen Lacklösemittel wie z.B. Ethylacetat, Butylacetat, Ethylenglykolmonomethyl- oder -ethyletheracetat, 1-Methoxypropyl-2-acetat, 2-Butanon, 4-Methyl-2-pentanon, Cyclohexanon, Toluol, Xylol, Solventnaphtha oder deren Gemische. Ebenfalls geeignet sind jedoch auch Lösemittel wie N-Methylpyrrolidon oder N-Methylcaprolactam oder Weichmacher wie z.B. solche auf Basis von Phosphorsäure-, Sulfonsäure- oder Phthalsäureester.

Die Anstrich- und Überzugsmittel können in Lösung oder aus der Schmelze oder in fester Form nach den üblichen Methoden wie z.B. Streichen, Rollen, Gießen, Spritzen, dem Wirbelsinterverfahren oder dem elektrostatischen Pulversprühverfahren auf den zu beschichtenden Gegenstand aufgebracht werden.

Der wesentliche Vorteil der erfindungsgemäßen Verfahrensprodukte ist darin zu sehen, daß durch die geeignete Wahl des molaren Verhältnisses der beiden Diisocyanate in der Ausgangsmischung die Eigenschaften der Verfahrensprodukte dem jeweiligen Anwendungszweck optimal angepaßt werden können. So kombinieren beispielsweise HDI/IPDI-Mischdimerisate mit hohem HDI-Gehalt den Vorteil der niedrigen Viskosität des reinen HDI-Uretdions mit der guten Härte der aus cycloaliphatischen Dimerisaten hergestellten Lackfilme. Andererseits weisen HDI/IPDI-Mischdimerisate mit hohem IPDI-Anteil die hohen Viskositäten des cycloaliphatischen Uretdions auf, was sie vor allem in blockierter Form für den Einsatz in Pulverlacken, die bevorzugt nach dem elektrostatischen Sprühverfahren aufgebracht werden, prädestiniert; gleichzeitig zeigen die aus ihnen formulierten Lacke eine durch den HDI-Anteil verursachte gewisse Flexibilität.

Die folgenden Beispiele dienen zur weiteren Erläuterung der Erfindung. Alle Prozentangaben beziehen sich, soweit nichts anderslautendes vermerkt, auf Gewichtsprozente.

Der prozentuale Anteil der Uretdiongruppen im Verfahrensprodukt wurde durch Heißtitration des NCO-Gehaltes (3 h Rückfluß in o-Dichlorbenzol) ermittelt. Der Gehalt an Isocyanuratgruppen wurde aus Art und Menge der Ausgangsmaterialien, sowie dem Isocyanat- und Uretdion-Gehalt des Verfahrensprodukts errechnet. Der IPDI-Anteil im Verfahrensprodukt wurde ebenfalls aus der Zusammensetzung der Ausgangsmaterialien und des abdestillierten Diisocyanat-Überschusses errechnet. In einigen Beispielen wurde außerdem noch der Gehalt an Uretdiondiisocyanat und an Triisocyanatomonoisocyanurat (Mol.-%) gelchromatographisch bestimmt.

Beispiele

Beispiel 1

In ein Gemisch von 1512 g (9 Mol) HDI und 222 g (1 Mol) IPDI rührt man bei 50 °C nacheinander 17,3 g 2,2,4-Trimethyl-1,3-pentandiol (TMPD) und 5,2 g Tri-n-octylphosphin ein und erwärmt anschließend auf 60 °C. Nach einer Reaktionszeit von 7 h ist der NCO-Gehalt der Mischung auf 38,5 % abgesunken. Die Reaktion wird durch Zugabe von 2,6 g p-Toluolsulfonsäuremethylester (TSE) und einstündiges Erhitzen auf 80 °C abgestoppt. Nach Abdestillieren des nicht umgesetzten Diisocyanatgemisches im Dünnschichtverdampfer bei 140 °C und einem Druck von 0,05 mbar erhält man das praktisch farblose Dimerisierungsprodukt mit einem NCO-Gehalt von 20,7 % und einer Viskosität von 270 mPa.s (23 °C). Der Gehalt an freiem HDI beträgt 0,16 %, an freiem IPDI 0,17 %. Aus der Zusammensetzung des zurückgewonnenen Diisocyanatgemisches läßt sich für das Umsetzungsprodukt rechnerisch ein IPDI-Anteil von 7 Mol-% ermitteln. Die Heißtitration des NCO-Gehaltes ergibt einen Uretdiongruppengehalt von 12,4 %. Das IR-Spektrum des Produktes zeigt bei 2272 und 1767 $cm^{-1}$ intensive Banden für Isocyanat- und Urethdiongruppen, während die für Isocyanurate charakteristische Bande bei 1690 $cm^{-1}$ nur eine untergeordnete Bedeutung besitzt.

Beispiele 2 bis 9

Die Umsetzungen wurden wie in Beispiel 1 beschrieben durchgeführt. Die Zusammensetzung der Ausgangsgemische, Reaktionsparameter sowie charakteristischen Daten der Produkte sind in Tabelle 1 aufgelistet.

Tabelle 1

| Beispiel | 2 | 3 | 4 | 5 | 6 | 7 | 8 | 9 |
|---|---|---|---|---|---|---|---|---|
| **Ausgangsmischung** | | | | | | | | |
| HDI (g) | 1000 | 1000 | 800 | 454 | 267 | 267 | 202 | 67 |
| IPDI (g) | 440 | 440 | 1050 | 1400 | 1407 | 1407 | 1510 | 799 |
| HDI:IPDI (Mol) | 3:1 | 3:1 | 1:1 | 3:7 | 1:4 | 1:4 | 15:85 | 1:9 |
| Katalysator[a)] | TBP | TOP | TBP | TOP | TBP | TOP | TOP | TBP |
| **Reaktion** | | | | | | | | |
| Reaktionszeit (h) | 4,5 | 8 | 6 | 20 | 26 | 22 | 48 | 48 |
| % NCO Rohware | 39,3 | 35,7 | 37,3 | 35,4 | 32,9 | 32,3 | 32,4 | 32,7 |
| **Destillation** | | | | | | | | |
| Temperatur (°C) | 135 | 135 | 135 | 140 | 135 | 140 | 135 | 135 |
| Druck (mbar) | 0,04 | 0,01 | 0,01 | 0,07 | 0,01 | 0,01 | 0,07 | 0,01 |
| **Harz** | | | | | | | | |
| % NCO | 20,7 | 20,5 | 19,3 | 17,8 | 18,1 | 17,7 | 17,6 | 17,4 |
| Viskosität (mPa.s/23°C) | 375 | 500 | 1680 | 25400 | 120000 | >150000 | >200000 | >200000 |
| freies HDI (%) | 0,17 | 0,10 | 0,09 | 0,11 | 0,04 | 0,07 | 0,09 | 0,03 |
| freies IPDI (%) | 0,13 | 0,15 | 0,31 | 0,28 | 0,11 | 0,37 | 0,39 | 0,42 |
| Uretdiongehalt (%) | 16,6 | 16,4 | 13,9 | 12,7 | 12,7 | 14,1 | 13,7 | 13,6 |
| Isocyanuratgehalt (%) | 10,5 | 10,3 | 11,9 | 11,8 | 10,2 | 9,2 | 9,8 | 9,5 |
| IPDI-Anteil (Mol-%) | 18 | 23 | 40 | 63 | 74 | 74 | 73 | 78 |
| Uretdiondiisocyanat (%) | 41,5 | - | - | 36,8 | - | 31,5 | 43,7 | - |
| Triisocyanatomonoisocyanuart (%) | 22,9 | - | - | 21,9 | - | 22,6 | 24,6 | - |
| höhere Homologe (%) | 35,6 | - | - | 41,3 | - | 45,9 | 31,7 | - |
| **IR ($cm^{-1}$)** | | | | | | | | |
| NCO | 2270 | 2271 | 2268 | 2261 | 2261 | 2261 | 2259 | 2257 |
| Uretdion | 1766 | 1767 | 1767 | 1767 | 1767 | 1767 | 1766 | 1766 |
| Isocyanurat | 1690 | 1690 | 1691 | 1692 | 1692 | 1692 | 1691 | 1693 |

Erläuterung zur vorstehenden Tabelle:

a) TBP = Tri-n-butylphosphin;
TOP = Tri-n-octylphosphin
Jeweils 0,3 Gew.-%, bezogen auf die Gesamtmenge an eingesetztem Diisocyanat.
Als Co-Katalysator jeweils 1 Gew.-% TMPD, bezogen auf die Gesamtmenge an eingesetztem Diisocyanat. Als Stopper TSE in äquimolarer Menge, bezogen auf den jeweils eingesetzten Katalysator.

Vergleichsbeispiel

1000 g (4,5 Mol) IPDI werden bei 50 °C nacheinander mit 10 g TMPD und 3 g TBP versetzt, anschließend auf 60 °C erwärmt und bei dieser Temperatur gehalten. Der NCO-Gehalt der Reaktionsmischung sinkt innerhalb von 24 h von anfangs 37,8 % auf 36,5 % ab und blieb während weiterer 48 h konstant. Der Dimerisierungsgrad beträgt 1,35 %.

## Patentansprüche

1. Polyisocyanatgemische mit einem Uretdiongehalt (berechnet als $C_2N_2O_2$) von 8 bis 25 Gew.-% und einem Isocyanuratgehalt (berechnet als $C_3N_3O_3$) von max. 24 Gew.-%, bestehend im wesentlichen aus

   a) 20 bis 60 Gew.-% Uretdiondiisocyanat der Formel (I)

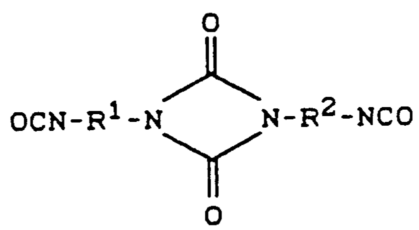

,

   b) 0 bis 40 Gew.-% Isocyanurattriisocyanat der Formel (II)

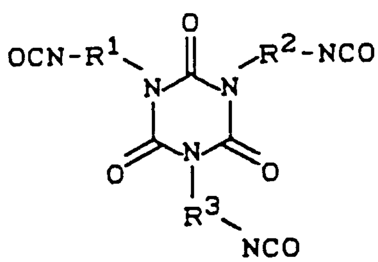

,

   c) 15 bis 60 Gew.-% an höheren, mehr als einen Uretdion- und/oder Isocyanuratring aufweisenden Homologen der Polyisocyanate I und/oder II und

   d) insgesamt maximal 1 Gew.-% 1,6-Diisocyanatohexan (HDI) und 1-Isocyanato-3,3,5-trimethyl-5-isocyanatomethyl-cyclohexan (IPDI),

   wobei sich die genannten Prozentsätze zu 100 ergänzen, und wobei

   $R^1$, $R^2$ und $R^3$ gleich oder verschieden sind und für die die Isocyanatgruppen von HDI oder IPDI verknüpfenden Kohlenwasserstoffreste stehen, wobei im letztgenannten Fall die Isocyanatgruppen mit dem cycloaliphatischen Ring verknüpft sind, mit der Maßgabe, daß mindestens 15 Mol-% und höchstens 95 Mol-% der genannten Reste Hexamethylenreste darstellen.

2. Verwendung des Polyisocyanatgemischs gemäß Anspruch 1, gegebenenfalls in mit Blockierungsmitteln für Isocyanatgruppen blockierter Form, als Isocyanatkomponente in Polyurethanlacken.

## Claims

1. Polyisocyanate mixtures with a uretdione content (expressed as $C_2N_2O_2$) of 8 to 25% by weight and an isocyanurate content (expressed as $C_3N_3O_3$) of at most 24% by weight, consisting essentially of

   a) 20 to 60% by weight of uretdione diisocyanate corresponding to formula (I):

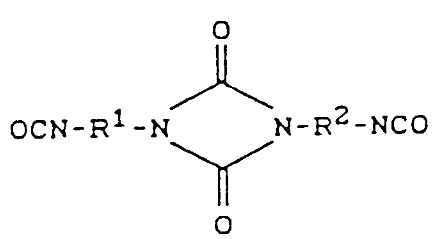

,

b) 0 to 40% by weight of isocyanurate triisocyanate corresponding to formula (II):

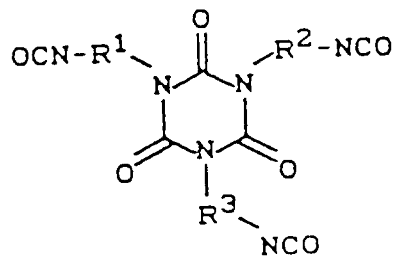

,

c) 15 to 60% by weight of higher homologs of polyisocyanates I and/or II containing more than one uretdione and/or isocyanurate ring and

d) a total of at most 1% by weight of 1,6-diisocyanatohexane (HDI) and 1-isocyanato-3,3,5-trimethyl-5-isocyanatomethyl cyclohexane (IPDI),

the percentages mentioned adding up to 100 and $R^1$, $R^2$ and $R^3$ in the above formulae being the same or different and representing the hydrocarbon radicals linking the isocyanate groups of HDI or IPDI, the isocyanate groups in the last-mentioned case being attached to the cycloaliphatic ring, with the proviso that at least 15 mole-% and at most 95 mole-% of the radicals mentioned are hexamethylene radicals.

**2.** The use of the polyisocyanate mixture claimed in claim 1, optionally blocked with blocking agents for isocyanate groups, as isocyanate component in polyurethane paints.

## Revendications

**1.** Mélanges de polyisocyanates à une teneur en uret-diones (exprimée en $C_2N_2O_2$) de 8 à 25 % en poids et une teneur en isocyanurates (exprimée en $C_3N_3O_3$) de 24 % en poids au maximum, qui consiste essentiellement en

a) 20 à 60 % en poids d'uret-dione-diisocyanate de formule (I):

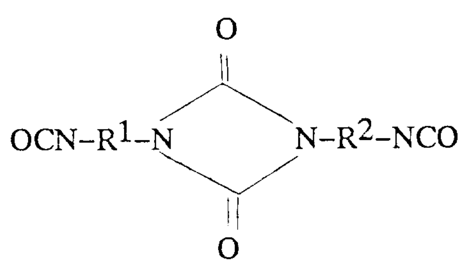

,

b) 0 à 40 % en poids d'isocyanurate-triisocyanate de formule (II):

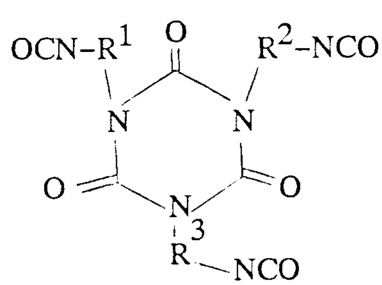

,

c) 15 à 60 % en poids d'homologues supérieurs, contenant plus d'un cycle uretdione et/ou isocyanurate, des polyisocyanates I et/ou II et

d) au total 1 % en poids au maximum de 1,6-diisocyanatohexane (HDI) et de 1-isocyanato-3,3,5-triméthyl-5-isocyanatométhyl-cyclohexane(IPDI),

les pourcentages indiqués représentant 100 au total, et

$R^1$, $R^2$ et $R^3$, ayant des significations identiques ou différentes, représentent les radicaux hydrocarbonés reliant les groupes isocyanates du HDI ou de l'IPDI, les groupes isocyanates étant reliés dans ce dernier cas avec le noyau cycloaliphatique, sous réserve qu'au moins 15 mol % et au maximum 95 mol % des radicaux en question sont des radicaux hexaméthylène.

**2.** Utilisation des mélanges de polyisocyanates selon la revendication 1, éventuellement à l'état bloqué par des agents bloquants des groupes isocyanates, en tant que composants isocyanates dans des vernis de polyuréthanne.